# EUROPÄISCHE PATENTANMELDUNG

(11) **EP 1 600 179 A2**
(43) Veröffentlichungstag der Anmeldung: **30.11.2005**
(21) Anmeldenummer: 05103649.9
(22) Anmeldetag: 02.05.2005
(51) Int. Cl.: A61L 31/02, A61L 31/14

(54) **Implantat zur Gefässligatur**

(30) Priorität: 25.05.2004 DE 102004026104
(71) Anmelder: Restate Patent AG, 6340 Baar/Zug (CH)
(72) Erfinder: Gerold, Bodo Dr., 91225 Zellingen (DE); Harder, Claus Dr., 91080, Uttenreuth (DE); Heublein, Bernd Prof. Dr., verstorben (DE); Müller, Heinz Dr., 91054, Erlangen (DE)
(74) Vertreter: Eisenführ, Speiser & Partner

(57) **Zusammenfassung**

Die Erfindung betrifft u.a. ein Implantat zur Gefäßligatur bestehend zumindest in Teilen oder ganz aus einer biodegradierbaren Legierung folgender Zusammensetzung:

| | |
|---|---|
| Magnesium | > 87 % |
| Yttrium | 3%-6% |
| Lanthanoide | 1 % - 5 % |
| Rest | 0 % bis 2,0 %. |

## Beschreibung

Die Erfindung betrifft ein Implantat zur Gefäßligatur.

Unter Gefäßligatur wird in der Medizintechnik das am Ort der Wahl bzw. einer auch operativen Verletzung erfolgende Unter- oder Abbinden eines Blutgefäßes verstanden. Gegebenenfalls umfasst die Gefäßligatur auch die Durchstechung und Umstechung des Gefäßes. Zur Durchführung bedient sich der Operateur verschiedener Hilfsmittel, die u.a. auch im Körper des Patienten verbleibende Implantate umfassen. Beispielhaft für derartige Implantate zur Gefäßligatur sei die Behandlung von Aneurismen einerseits mittels einer Metallklammer (Clip) oder andererseits durch Platzierung eines Coils im Inneren des Gefäßes genannt.

Aus dem Stand der Technik bekannte Implantate zur Gefäßligatur bestehen in der Regel aus biokompatiblen, aber nicht biodegradierbaren Werkstoffen. Als Werkstoffe eignen sich insbesondere Kunststoffe und Metalle nebst ihrer Legierungen. Trotz aller Anstrengungen die Verträglichkeit der Implantate zu verbessern, ist die dauerhafte Anwesenheit der Implantate im menschlichen oder tierischen Körper oft Ausgangspunkt für Komplikationen, wie chronische Entzündungen.

Weiterhin ist die Kernspinresonanztomographie mittlerweile tägliche Routine in der radiologischen Diagnostik. Der Patient wird dabei sowohl einem starken statistischen als auch veränderlichen kleineren Magnetfeldern ausgesetzt. Bei Anwesenheit von Implantaten aus ferromagnetischen Materialien werden diese durch das Magnetfeld angezogen, bewegt und schlimmstenfalls aus ihrer ursprünglichen Position entfernt. Durch die Metalle können zudem erhebliche Bildfehler (Artefakte) auf den Schnittbildern einer Untersuchung entstehen. Je nach Umfang dieser Artefakte kann die Beantwortung der anstehenden diagnostischen Fragestellung erschwert oder in extremen Fall sogar völlig unmöglich gemacht werden. Implantate aus Kunststoffe mögen zwar diese Effekte ausschließen, haben aber zumeist für die Applikation ungeeignete Werkstoffeigenschaften.

Häufig ist die Anwesenheit der Implantate aus medizinischer Sicht nur für einen begrenzten Zeitraum notwendig, sei es durch das natürliche und dauerhafte Zuwachsen des Gefäßes nach dessen Verschluss oder sei es durch Abschluss des Heilungsprozesses in dem nur für kurze Zeit verschlossenen Teil des Blutgefäßes. Das in einem solchen Fall überflüssig gewordene Implantat kann nur durch erneute Operation entfernt werden.

Aus der DE 101 28 100 ist ein medizinisches Implantat für den menschlichen und tierischen Körper bekannt, dass zumindest teilweise aus einer biodegradierbaren Magnesiumlegierung besteht, die Anteile von Seltenerdmetallen und Lithium enthält. Es wird unter anderem die Verwendung als chirurgisches Nahtmaterial, insbesondere Wundklammern, angesprochen. Die Magnesiumlegierung enthält vorzugsweise Anteile an Lithium von 0-7 %, Aluminium von 0-16 %, Seltenerdmetallen von 0-8 % und Yttrium von 0-7 %. Das Seltenerdmetall kann Neodym sein.

Weiterhin sind aus der EP 1 170 023 Coils und Clips bekannt, die aus einem in vivo abbaubaren, metallischen Werkstoff bestehen. Der metallische Werkstoff ist eine Legierung, deren Hauptbestandteil ein Erdalkalimetall, insbesondere Magnesium, sein kann.

Ein für den Einsatz in Implantaten zur Gefäßligatur geeigneter biodegradierbarer Werkstoff sollte mehreren Erfordernissen genügen:
- Er sollte die für Herstellung und Verwendung des Implantats notwendigen mechanischen Eigenschaften besitzen (Verformbarkeit, Bruchfestigkeit, etc.),
- der Werkstoff selbst, als auch seine Abbauprodukte sollten physiologisch unbedenklich sein und
- der in vivo Abbau sollte gleichmäßig und kontrolliert ablaufen sowie der jeweiligen Applikation angepasst sein.

Die Suche nach einem geeigneten Werkstoff ist entsprechend aufwendig. Alle vorbekannten Lösungen haben bisher nicht zu einem befriedigenden Ergebnis geführt.

Demnach liegt der vorliegenden Erfindung unter anderem die Aufgabe zugrunde einen für Implantate zur Gefäßligatur geeigneten Werkstoff zu Verfügung zu stellen.

Es hat sich nun überraschenderweise gezeigt, dass Implantate zur Gefäßligatur, die zumindest in Teilen oder ganz aus einer biodegradierbaren Legierung folgender Zusammensetzung bestehen:

| | |
|---|---|
| Magnesium | > 87 %, |
| Yttrium | 3 % - 6 %, |
| Lanthanoide | 1 % - 5 %, |
| Rest | 0 % - 2 %, |

besonders gute Werkstoffeigenschaften für den genannten Einsatzzwecke haben und die Abbauprodukte sogar einen positiven physiologischen Effekt auf das umgebende Gewebe besitzen. Alle Angaben zur Legierung sind auf Gewichtsprozente bezogen, wobei sich die einzelnen Legierungskomponenten zu 100 % aufsummiert.

Ausgesprochen gute Eigenschaften des Werkstoffs sowie vielversprechende physiologische Effekte der Abbauprodukte lassen sich vorzugsweise einerseits mit einer Legierung mit einem Yttrium-Anteil von 3,7 - 4,3 % und einem Lanthanoid-Anteil von 2,4 - 4,4 % und andererseits mit einer Legierung mit einem Yttrium-Anteil von 4,75 - 5,5 % und einem Lanthanoid-Anteil von 1,5 - 4 % erzielen.

Ferner ist bevorzugt, dass der Lanthanoid-Anteil als Hauptbestandteil das Element Neodym umfasst. Es hat sich überraschenderweise gezeigt, dass ein Neodym-Anteil, insbesondere in Höhe von 2 - 2,5 % an der Legierung, die physiologische Verträglichkeit der Legierung und seiner Abbauprodukte verbessert. Zudem zeigt die Legierung für die Herstellung und Verwendung von Implantaten zur Gefäßligatur, insbesondere von Clips und Coils, besonders geeignete Werkstoffeigenschaften, wie nicht-ferromagnetisches Verhalten, leichte Verformbarkeit und ausreichende Bruchfestigkeit.

Weiterhin ist bevorzugt, dass der Rest eines oder mehrere der Elemente aus der Gruppe Lithium, Zirkonium und Zink umfasst, wobei vorzugsweise ein Lithium-Anteil bei 0,15 - 2 %, ein Zirkonium-Anteil bei 0,4 - 1 % und ein Zink-Anteil bei 0,004 - 0,2 % liegt. Die Anwesenheit der genannten Elemente für sich genommen oder in beliebiger Kombination hat offenbar Einfluss auf die mechanischen Eigenschaften des Implantats. So ist insbesondere eine in vivo Degradation des Implantates auch von dem vorgegebenen Lithium-Anteil abhängig. Des weiteren führt die Anwesenheit von Zirkonium zu einer deutlichen Minderung der Spannungs-Riss-Korrosion.

Ein Degradationsverhalten des Implantats kann vorzugsweise in Abhängigkeit von einer Legierungsmorphologie, einer Materialstärke des Implantats sowie der Legierungszusammensetzung vorgegeben werden. Unter dem Begriff "Degradationsverhalten" wird der über die Zeit durch chemische, thermische, oxidative, mechanische oder biologische Prozesse stattfindende Abbau der erfindungsgemäßen Polysaccharidschicht im lebendem Organismus verstanden. Einerseits soll sichergestellt werden, dass zumindest in den ersten Wochen nach der Implantation die gewünschten medizin-technischen Eigenschaften des Implantates, die in der Regel durch seine mechanische Integrität bestimmt sind, bestehen bleiben. Andererseits soll die Anwesenheit von starren Strukturen, die Ausgangspunkt einer Kaskade von Abstoßungsreaktionen sein können, nur über einen unbedingt notwendigen Zeitraum aufrecht erhalten werden.

Vorzugsweise wird das Degradationsverhalten des Implantats derart vorgegeben, dass 80 Gew.-% oder mehr des Implantats, bezogen auf das Gesamtgewicht der im Implantat vorhandenen Legierung, in einem Zeitraum zwischen 6 Monaten bis 10 Jahren abgebaut sind. Ein weiterer Aspekt der Erfindung besteht darin, dass das Degradationsverhalten des Implantats derart vorzugeben ist, dass seine mechanische Integrität für mindestens 4, insbesondere 6 Monate, aufrecht erhalten bleibt. Unter "mechanischer Integrität" wird dabei die trotz fortschreitendem Abbau noch ausreichende Stabilität der Strukturelemente des Implantates verstanden, die zur Erfüllung des medizinischen Zwecks des Implantats, d.h. Unter- oder Abbinden eines Gefäßes, notwendig sind. Mit anderen Worten, die Degradation kann demnach zwar bereits zum Abbau eines erheblichen, aber eben nicht zur Wahrung des medizinischen Zwecks notwendigen Teils des Implantates geführt haben.

Die Degradation des Implantats im vorgenannten Sinne kann beispielsweise durch Erhöhung der Materialstärke verzögert werden. Ebenso haben herstellungsbedingte Verfahrensschritte Einfluss auf die Degradation (so degradieren gegossene Implantate in der Regel schneller als stranggepresste). Weiterhin führt eine Erhöhung eines Lithium-Anteils zu einem verzögerten Abbau. Aufgrund der Komplexität des in vivo Abbaus, der nicht nur von der Formgebung des Implantats und der Morphologie und Zusammensetzung des eingesetzten Material abhängt, sondern auch von der Lage des Implantats im Körper abhängt, ist jeweils eine fallbezogene Bestimmung des Degradationsverhaltens eines für bestimmte Zwecke vorgesehenen Implantats notwendig.

Es hat sich weiter überraschenderweise gezeigt, dass stranggepresste Legierungen im Vergleich zu gegossenen Legierungen verbesserte physiologische Eigenschaften besitzen. Die physiologischen Eigenschaften sind somit zumindest zum Teil durch den Herstellungsprozess bedingt. So zeigten herkömmliche Zelltests an unbehandelten glatten, humanen Muskelzellen eine ausgeprägte Proliferationshemmung in Gegenwart der erfindungsgemäßen Legierung und seiner Abbauprodukte. Der genaue physiologische Wirkmechanismus ist bisher nicht geklärt.

Der nachfolgenden Tabelle lassen sich zwei Beispiele für zur Herstellung von Clips oder Coils geeignete Legierungen entnehmen:

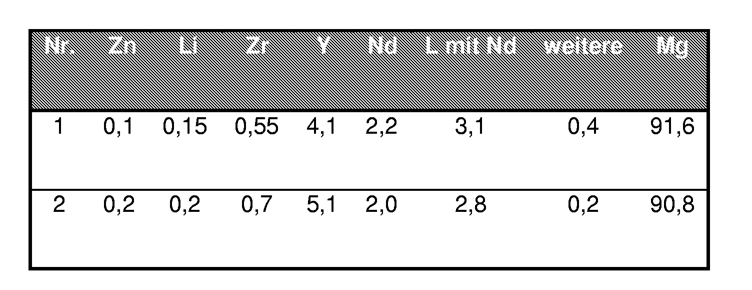

Die Angaben zu den Legierungskomponenten beziehen sich auf Gewichtsprozente. 'L' steht für Lanthanoide und 'weitere' für andere in der Legierungskomponente Rest zusammengefasste Elemente, wie Silizium, Kupfer, Mangan, Eisen, Nickel und Silber. Die Angaben wurden mit einer Genauigkeit von etwa +/- 0,1 % bestimmt.

## Patentansprüche

1. Implantat zur Gefäßligatur bestehend zumindest in Teilen oder ganz aus einer biodegradierbaren Legierung folgender Zusammensetzung:
| | |
|---|---|
| Magnesium | > 87 % |
| Yttrium | 3 % - 6 % |
| Lanthanoide | 1 % - 5 % |
| Rest | 0 % bis 2,0 %. |

2. Implantat nach Anspruch 1, **dadurch gekennzeichnet, dass** der Yttrium-Anteil der Legierung 3,7 - 4,3 % und der Lanthanoid-Anteil der Legierung 2,4 - 4,4 % beträgt.

3. Implantat nach Anspruch 1, **dadurch gekennzeichnet, dass** der Yttrium-Anteil der Legierung 4,75 - 5,5 % und der Lanthanoid-Anteil der Legierung 1,5 - 4,0 % beträgt.

4. Implantat nach einem oder mehreren der Ansprüche 1 bis 3, **dadurch gekennzeichnet, dass** der Lanthanoid-Anteil als Hauptbestandteil Neodym umfasst.

5. Implantat nach Anspruch 4, **dadurch gekennzeichnet, dass** der Neodym-Anteil der Legierung 2,0 - 2,5 % beträgt.

6. Implantat nach einem oder mehreren der Ansprüche 1 bis 5, **dadurch gekennzeichnet, dass** der Rest eines oder mehrere der Elemente aus der Gruppe Lithium, Zirkonium und Zink umfasst.

7. Implantat nach Anspruch 6, **dadurch gekennzeichnet, dass** der Lithium-Anteil der Legierung 0,15 - 0,2 % beträgt.

8. Implantat nach Anspruch 6, **dadurch gekennzeichnet, dass** der Zirkonium-Anteil der Legierung 0,4 - 1,0 % beträgt.

9. Implantat nach Anspruch 6, **dadurch gekennzeichnet, dass** der Zink-Anteil der Legierung 0,004 - 0,2 % beträgt.

10. Implantat nach einem oder mehreren der Ansprüche 1 bis 9, **dadurch gekennzeichnet, dass** das Implantat ein Clip oder ein Coil ist.

11. Implantat nach einem oder mehreren der Ansprüche 1 bis 10, **dadurch gekennzeichnet, dass** ein Degradationsverhalten des Implantats so vorgegeben ist, dass 80 Gew.% oder mehr des Implantats, bezogen auf das Gesamtgewicht der im Implantat vorhandenen Legierung, in einem Zeitraum zwischen 6 Monaten bis 10 Jahren abgebaut sind.

12. Implantat nach einem oder mehreren der Ansprüche 1 bis 11, **dadurch gekennzeichnet, dass** ein Degradationsverhalten des Implantats so vorgegeben ist, dass eine mechanische Integrität für mindestens 4, insbesondere 6 Monate, aufrecht erhalten bleibt.
